(19) Europäisches Patentamt
European Patent Office
Office européen des brevets

(11) Veröffentlichungsnummer: **0 024 018**
**B1**

# (12) EUROPÄISCHE PATENTSCHRIFT

(45) Veröffentlichungstag der Patentschrift: **06.07.83**

(51) Int. Cl.³: **C 07 F 9/40**

(21) Anmeldenummer: **80104526.1**

(22) Anmeldetag: **31.07.80**

(54) **Verfahren zur Herstellung von alpha-Oxo-alpha-(3-alkoxycarbonyl-2,2-dimethyl-cycloprop-1-yl)-methan-phosphonsäureestern, neue Zwischenprodukte hierfür und Verfahren zu deren Herstellung.**

(30) Priorität: **09.08.79 DE 2932262**

(43) Veröffentlichungstag der Anmeldung: **18.02.81 Patentblatt 81/7**

(45) Bekanntmachung des Hinweises auf die Patenterteilung: **06.07.83 Patentblatt 83/27**

(84) Benannte Vertragsstaaten: **BE CH DE FR GB IT LI NL**

(56) Entgegenhaltungen:
**JOURNAL OF ORGANIC CHEMISTRY Band 43, Nr. 5, 03. März 1978 Easton, USA, J. R. REID et al. »Synthesis of Methyl Arylmethyl 2,2-Dimethyl-3-(2-methyl-1-propenyl)cyclopropylphosphonates as Potential Insecticides« Seiten 999 bis 1000**
**Chemical Abstracts Band 77, Nr. 17, 23. Oktober 1972 Columbus, Ohio, USA D. W. GIER »Dialkenyl esters of aryloxyacetyl phosphonic acids as herbicides« Seite 133, linke Spalte, Abstract Nr. 110 570 h**
**Chemical Abstracts Band 71, August 1969 Columbus, Ohio, USA A. W. JOHNSON et al. »Chemistry of ylides. XIX beta-Carbonyl sulfonium ylides« Seite 290, linke Spalte, Abstract Nr. 21 795u**

(73) Patentinhaber: **BAYER AG, Zentralbereich Patente, Marken und Lizenzen, D-5090 Leverkusen 1, Bayerwerk (DE)**

(72) Erfinder: **Hoffmann, Hellmut, Prof.Dr., Tersteegenweg 17, D-5600 Wuppertal 1 (DE)**

**Die Akte enthält technische Angaben, die nach dem Eingang der Anmeldung eingereicht wurden und die nicht in dieser Patentschrift enthalten sind.**

**EP 0 024 018 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Verfahren zur Herstellung von α-Oxo-α-(3-alkoxycarbonyl-2,2-dimethyl-cycloprop-1-yl)-methan-phosphonsäureestern, neue Zwischenprodukte hierfür und Verfahren zu deren Herstellung**

Die Erfindung betrifft ein neues Verfahren zur Herstellung von α-Oxo-α-(3-alkoxycarbonyl-2,2-dimethyl-cycloprop-1-yl)-methan-phosphorsäureestern, welche als Zwischenprodukte zur Herstellung von Pestiziden verwendet werden können, neue Zwischenprodukte hierfür und ein Verfahren zu deren Herstellung.

α-Oxo-α-(3-alkoxycarbonyl-2,2-dimethyl-cycloprop-1-yl)-methan-phosphonsäureester sind Gegenstand von noch nicht zum Stand der Technik gehörenden Anmeldungen der Anmelderin DE-OS 2 917 620 und DE-OS 2 929 670.

Gegenstand der vorliegenden Erfindung sind

(1) ein Verfahren zur Herstellung von α-Oxo-α-(3-alkoxycarbonyl-2,2-dimethyl-cycloprop-1-yl)-methan-phosphonsäureestern der Formel I

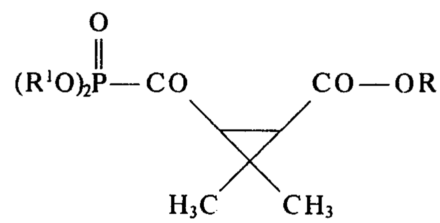

(I)

in welcher

R für Alkyl steht und
$R^1$ für Alkyl oder Phenyl steht oder die beiden Reste $R^1$ zusammen für geradkettiges oder verzweigtes Alkandiyl (Alkylen) stehen,

das dadurch gekennzeichnet ist, daß man 1-Oxo-3-methyl-2-butenphosphonsäureester der Formel II

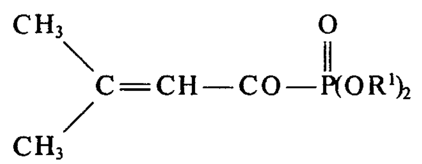

(II)

in welcher

$R^1$ die oben angegebene Bedeutung hat,

mit Sulfuranyliden-essigsäureestern der Formel III

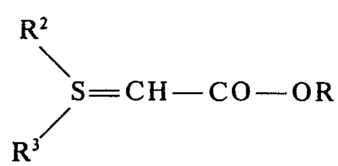

(III)

in welcher

R die oben angegebene Bedeutung hat und
$R^2$ und $R^3$ einzeln für Alkyl oder zusammen für Alkanidyl (Alkylen) stehen,

gegebenenfalls in Gegenwart eines Verdünnungsmittels bei Temperaturen zwischen 0 und 100°C umsetzt,

(2) neue 1-Oxo-3-methyl-2-buten-phosphonsäureester der Formel II

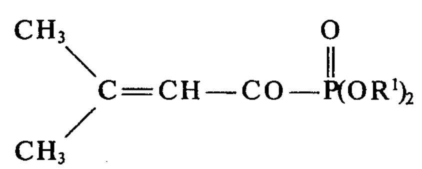

(II)

in welcher

$R^1$ für Alkyl oder Phenyl steht oder die beiden Reste $R^1$ zusammen für geradkettiges oder verzweigtes Alkandiyl (Alkylen) stehen;

(3) ein Verfahren zur Herstellung von 1-Oxo-3-methyl-2-buten-phosphonsäureestern der Formel II, das dadurch gekennzeichnet ist, daß man in an sich bekannter Weise β,β-Dimethyl-acrylsäurechlorid der Formel IV

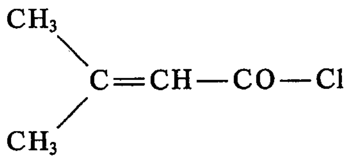

(IV)

mit Phosphorigsäureestern der Formel V

$$R^4O—P(OR^1)_2 \qquad (V)$$

in welcher

$R^1$ die oben angegebene Bedeutung hat und
$R^4$ für Methyl oder Ethyl steht,

gegebenenfalls in Gegenwart eines Verdünnungsmittels bei Temperaturen zwischen 0 und 100° C umsetzt.

Es ist als überraschend anzusehen, daß man nach dem erfindungsgemäßen Verfahren α-Oxo-α-(3-alkoxy-carbonyl-2,2-dimethyl-cycloprop-1-yl)-methan-phosphonsäureester in sehr hohen Ausbeuten erhält, da im allgemeinen bei Reaktionen von β,β-Dimethylacrylsäurederivaten mit Sulfuranyliden-essigsäureestern die entsprechenden Cyclopropanderivate nicht oder nur in geringen Ausbeuten gebildet werden.

Verwendet man als Ausgangsstoffe beispielsweise 1-Oxo-3-methyl-2-buten-phosphonsäure-dimethylester und Dimethylsulfuranyliden-essigsäure-methylester, so kann die erfindungsgemäße Reaktion dieser Verbindungen durch folgendes Formelschema skizziert werden:

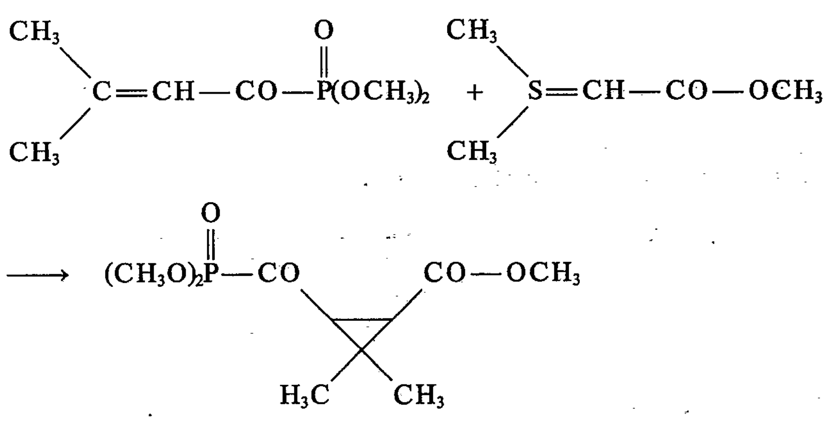

Das erfindungsgemäße Verfahren, welches oben unter (1) dargelegt ist (»Verfahren (1)«), wird vorzugsweise unter Verwendung von geeigneten Lösungs- oder Verdünnungsmitteln durchgeführt. Als solche kommen praktisch alle inerten organischen Solventien in Betracht. Hierzu gehören insbesondere aliphatische und aromatische, gegebenenfalls chlorierte Kohlenwasserstoffe, wie z. B. Pentan, Hexan, Heptan, Cyclohexan, Benzol, Toluol, Xylole, Methylenchlorid, Ethylenchlorid, Chloroform, Tetrachlorkohlenstoff, Chlorbenzol und Dichlorbenzole, Ether wie z. B. Diethyl- und Dibutylether, Tetrahydrofuran und Dioxan sowie Nitrile, wie z. B. Acetonitril und Propionitril.

Die Reaktionstemperatur wird bei Verfahren (1) im allgemeinen zwischen 0 und 100° C, vorzugsweise zwischen 20 und 80° C gehalten. Das Verfahren wird im allgemeinen bei Normaldruck durchgeführt.

Zur Durchführung von Verfahren (1) werden die Ausgangsverbindungen der Formeln (II) und (III) gewöhnlich in angenähert äquimolaren Mengen eingesetzt, d. h. das Molverhältnis der Komponenten liegt bei 1 zu 1±0,1. In einer bevorzugten Ausführungsform des Verfahrens werden die 1-Oxo-3-methyl-2-buten-phosphonsäureester, gegebenenfalls in einem der oben angegebenen

Verdünnungsmittel, vorgelegt und die Sulfuranyliden-essigsäureester dazugegeben. Die Mischung der Komponenten wird bis zum Ende der Umsetzung bei der oben angegebenen Temperatur gerührt; dann wird das Lösungsmittel sorgfältig im Vakuum abdestilliert, wobei das Produkt der Formel (I) als öliger Rückstand verbleibt.

Die als Ausgangsstoffe zu verwendenden neuen 1-Oxo-3-methyl-2-buten-phosphonsäureester sind durch Formel (II) definiert. Vorzugsweise steht darin

$R^1$ für $C_1-C_4$-Alkyl oder Phenyl oder die beiden Reste $R^1$ stehen zusammen für 2,2-Dimethyl-propan-1,3-diyl.

Als Beispiele seien 1-Oxo-3-methyl-2-buten-phosphonsäure-dimethylester, -diethylester und -diphenylester sowie 2-Oxo-2-(1-oxo-3-methyl-2-buten-1-yl)-5,5-dimethyl-1,3,2-dioxaphosphorinan genannt.

Man erhält die neuen 1-Oxo-3-methyl-2-buten-phosphonsäureester der Formel (II) nach dem oben unter (3) dargelegten Verfahren, indem man $\beta,\beta$-Dimethyl-acryl-säurechlorid der Formel IV (oben) mit Phosphorigsäureestern der Formel V (oben), wie z. B. mit Trimethyl- oder Triethylphosphit, 2-Methoxy- oder 2-Ethoxy-5,5-dimethyl-1,3,2-dioxaphosphorinan, gegebenenfalls in Gegenwart eines Verdünnungsmittels, wie z. B. Methylenchlorid (oder eines anderen der oben für Verfahren (1) angegebenen Lösungsmittel), bei Temperaturen zwischen 0 und 100°C, vorzugsweise zwischen 20 und 80°C, umsetzt und gegebenenfalls das Rohprodukt destilliert.

Als Ausgangsstoffe einzusetzende Phosphorigsäureester sind, ebenso wie $\beta,\beta$-Dimethyl-acrylsäurechlorid, bekannt.

Die bei Verfahren (1) als weitere Ausgangsstoffe zu verwendenden Sulfuranyliden-essigsäureester sind durch Formel (III) definiert.

Vorzugsweise stehen darin

R für $C_1-C_4$-Alkyl und
$R^2$ sowie $R^3$ einzeln für Methyl oder zusammen für Butan-1,4-diyl (Tetramethylen).

Als Beispiele für die Ausgangsstoffe der Formel (III) seien genannt:

Dimethylsulfuranyliden-essigsäure-methylester, -ethylester, -n-propylester, -iso-propylester, -n-butylester, -iso-butylester, -sek.-butylester und -tert.-butylester sowie
Tetramethylensulfuranyliden-essigsäure-methylester, -ethylester, -n-propylester, -iso-propylester, -n-butylester, -iso-butylester, -sek.-butylester und -tert.-butylester.

Verbindungen der Formel (III) sind bekannt (vergleiche J. Org. Chem. 32 [1967], 3351; US-PS 3 644 487 und DE-OS 2 724 734).

Aus den erfindungsgemäß herzustellenden $\alpha$-Oxo-$\alpha$-(3-alkoxycarbonyl-2,2-dimethyl-cycloprop-1-yl)-methanphosphonsäureestern der Formel (I) erhält man 3-Formyl-2,2-dimethyl-cyclopropan-1-carbonsäureester gemäß nachstehendem Formelschema durch Umsetzung mit Reduktionsmitteln, wie z. B. Natriumtetrahydridoborat (Natriumboranat), gegebenenfalls unter Verwendung von Verdünnungsmitteln, wie z. B. Wasser und/oder Methanol und/oder Methylenchlorid, bei Temperaturen zwischen −20 und +50°C, vorzugsweise zwischen −10 und +30°C und Umsetzung der hierbei erhaltenen $\alpha$-Hydroxy-$\alpha$-(3-alkoxycarbonyl-2,2-dimethyl-cycloprop-1-yl)-methan-phosphonsäureester mit wäßrigen Alkalilaugen, wie z. B. wäßriger Natronlauge, gegebenenfalls in Gegenwart eines mit Wasser nicht mischbaren Lösungsmittels, wie z. B. Methylenchlorid, bei Temperaturen zwischen 0 und 100°C, vorzugsweise zwischen 10 und 50°C (vergleiche Chem. Ber. 103 [1970], 2984—2986):

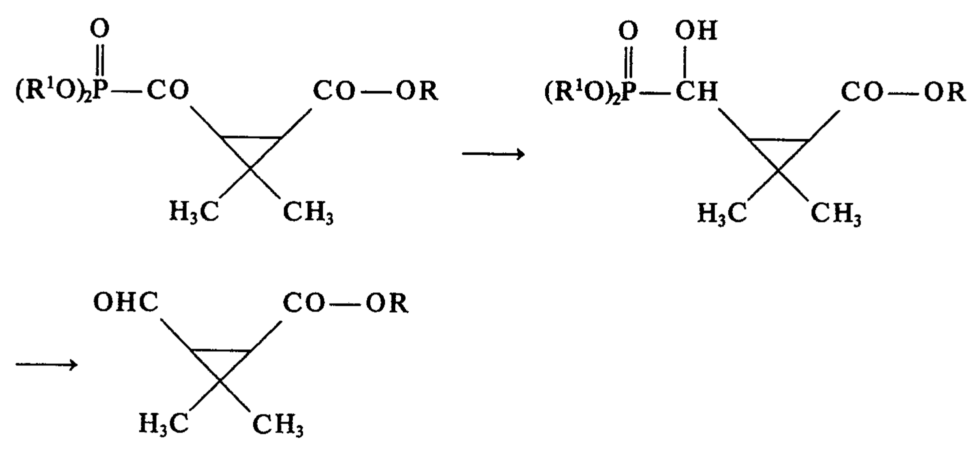

3-Formyl-2,2-dimethyl-cyclopropan-1-carbonsäureester können als Zwischenprodukte zur Herstellung von insektizid und akarizid wirksamen Pyrethroiden verwendet werden (vergleiche DE-OS 2 326 077).

Beispiel 1

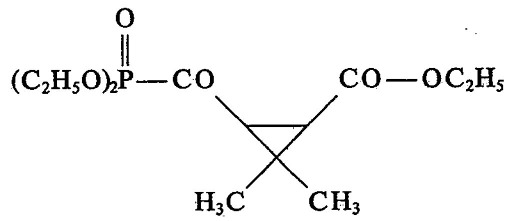

36 g (0,2 Mol) Tetramethylensulfuranyliden-essigsäureethylester werden innerhalb von 10 Minuten zu einer Lösung von 44 g (0,2 Mol) 1-Oxo-3-methyl-2-buten-phosphonsäure-diethylester in 150 ml Toluol gegeben. Die Innentemperatur steigt dabei auf ca. 55° C an. Das Reaktionsgemisch wird einige Stunden gerührt; dann wird das Lösungsmittel unter vermindertem Druck sorgfältig abdestilliert. Man erhält 58 g (95% der Theorie) α-Oxo-α-(3-ethoxycarbonyl-2,2-dimethyl-cycloprop-1-yl)-methan-phosphonsäure-diethylester als öligen Rückstand.

Elementaranalyse:
ber.: C 51,0%; H 7,5%; O 31,4%; P 10,1%;
gef.: C 50,4%; H 7,4%; O 31,3%; P 9,7%

Beispiel 2

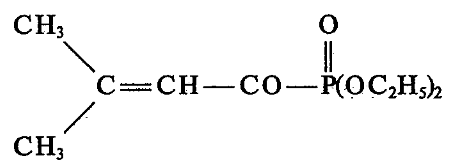

83 g (0,5 Mol) Triethylphosphit werden innerhalb einer Stunde zu einer Lösung von 60 g (0,5 Mol) β,β-Dimethylacrylsäurechlorid in 200 ml Methylenchlorid tropfenweise gegeben. Das Reaktionsgemisch wird noch eine Stunde bei 40 bis 45° C gerührt; dann wird das Lösungsmittel unter vermindertem Druck abdestilliert und das zurückbleibende Rohprodukt durch Vakuumdestillation gereinigt. Man erhält 90 g (82% der Theorie) 1-Oxo-3-methyl-2-buten-phosphonsäure-diethylester vom Siedepunkt 72° C/0,01 mbar.

Elementaranalyse:
ber.: C 49,1%; H 7,7%; O 29,1%; P 14,1%;
gef.: C 48,5%; H 7,6%; O 28,9%; P 13,4%.

Die als Ausgangsstoffe zu verwendenden Sulfuranylidenessigsäureester können beispielsweise wie folgt hergestellt werden:

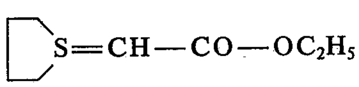

Eine Lösung von 12 g Kaliumhydroxid in 12 ml Wasser vermischt mit 55 ml gesättigter Kaliumcarbonatlösung wird zu einer auf 10 bis 15° C gekühlten, intensiv gerührten Lösung von 52 g (0,2 Mol) Tetramethylensulfoniumessigsäureethylester-bromid in 180 ml Chloroform gegeben. Das Reaktionsgemisch wird 15 Minuten bei 10° C gerührt und dann abgesaugt. Die beiden Phasen des Filtrats werden getrennt. Die Chloroformphase wird über Natriumsulfat getrocknet und nach Filtration wird das Lösungsmittel unter vermindertem Druck sorgfältig abdestilliert, wobei die Badtemperatur 30° C nicht überschreitet. Das zurückbleibende Produkt wird als Tetramethylensulfuranyliden-essigsäureethylester (100%ig) eingesetzt.

**Patentansprüche**

1. Verfahren zur Herstellung von α-Oxo-α-(3-alkoxycarbonyl-2,2-dimethyl-cycloprop-1-yl)-methan-phosphonsäureestern der Formel I

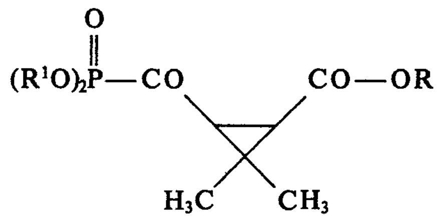

(I)

in welcher

R für Alkyl steht und
$R^1$ für Alkyl oder Phenyl steht oder die beiden Reste $R^1$ zusammen für geradkettiges oder verzweigtes Alkandiyl (Alkylen) stehen,

dadurch gekennzeichnet, daß man 1-Oxo-3-methyl-2-butenphosphonsäureester der Formel II

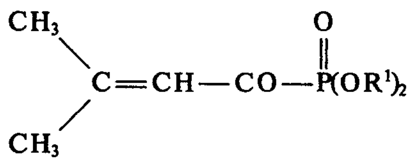

(II)

in welcher

$R^1$ die oben angegebene Bedeutung hat,

mit Sulfuranyliden-essigsäureestern der Formel III

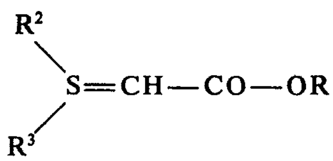

(III)

in welcher

R die oben angegebene Bedeutung hat und
$R^2$ und $R^3$ einzeln für Alkyl oder zusammen für Alkandiyl (Alkylen) stehen,

gegebenenfalls in Gegenwart eines Verdünnungsmittels bei Temperaturen zwischen 0 und 100°C umsetzt.

2. 1-Oxo-3-methyl-2-buten-phosphonsäureester der Formel II

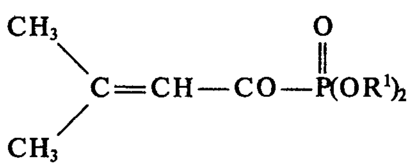

(II)

in welcher

$R^1$ für Alkyl oder Phenyl steht oder die beiden Reste $R^1$ zusammen für geradkettiges oder verzweigtes Alkandiyl (Alkylen) stehen.

3. Verfahren zur Herstellung von 1-Oxo-3-methyl-2-buten-phosphonsäureestern der Formel II, dadurch gekennzeichnet, daß man in an sich bekannter Weise β,β-Dimethyl-acrylsäurechlorid der Formel IV

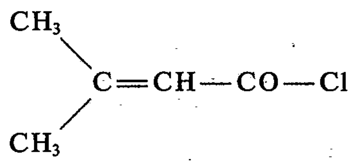

(IV)

mit Phosphorigsäureestern der Formel V

$$R^4O-P(OR^1)_2 \qquad (V)$$

in welcher

$R^1$ die oben angegebene Bedeutung hat und
$R^4$ für Methyl oder Ethyl steht,

gegebenenfalls in Gegenwart eines Verdünnungsmittels bei Temperaturen zwischen 0 und 100°C umsetzt.

## Claims

1. Process for the preparation of α-oxo-α-(3-alkoxycarbonyl-2,2-dimethyl-cycloprop-1-yl)-methane-phosphonic acid esters of the formula I

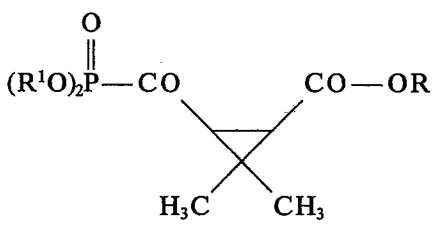

(I)

in which

R represents alkyl and
$R^1$ represents alkyl or phenyl, or the two radicals $R_1$ together represent straightchain or branched alkanediyl (alkylene),

characterised in that 1-oxo-3-methyl-2-butene-phosphonic acid esters of the formula II

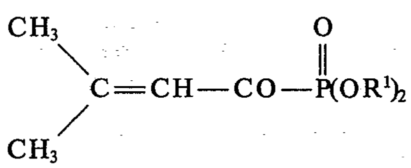

(II)

in which

$R^1$ has the meaning indicated above,

are reacted with sulphuranylidene-acetic acid esters of the formula III

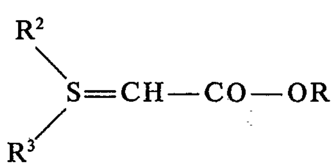

(III)

in which

R has the meaning indicated above and
$R^2$ and $R^3$ individually represent alkyl or together represent alkanediyl (alkylene),

if appropriate in the presence of a diluent, at temperatures between 0 and 100° C.

2. 1-Oxo-3-methyl-2-butene-phosphonic acid esters of the formula II

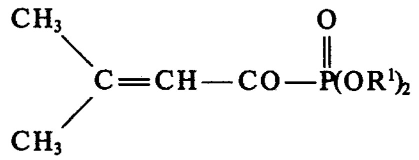

(II)

in which

$R^1$ represents alkyl or phenyl, or the two radicals $R^1$ together represent straightchain or branched alkanediyl (alkylene);

3. Process for the preparation of 1-oxo-3-methyl-2-butene-phosphonic acid esters of the formula II, characterised in that β,β-dimethyl-acrylic acid chloride of the formula IV

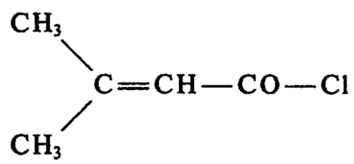

(IV)

is reacted with phosphorous acid esters of the formula V

$$R^4O—P(OR^1)_2 \quad (V)$$

in which

$R^1$ has the meaning indicated above and
$R^4$ represents methyl or ethyl,

in a manner known per se, if appropriate in the presence of a diluent, at temperatures between 0 and 100°C.

## Revendications

1. Procédé de fabrication d'esters d'acides α-oxo-α-(3-alcoxycarbonyl-2,2-diméthylcycloprop-1-yl)-méthane-phosphoniques de formule I:

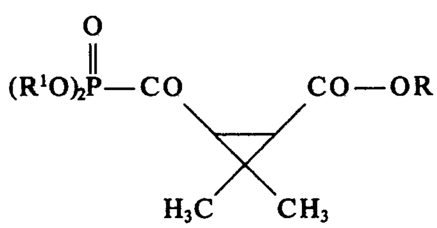

(I)

dans laquelle

R représente un alcoyle et
$R^1$ un alcoyle ou phényle ou bien les deux radicaux $R^1$ représentent ensemble un alcanediyle (alcoylène) à chaîne droite ou ramifiée,

caractérisé en ce qu'on fait réagir des esters d'acide 1-oxo-3-méthyl-2-butène-phosphonique de formule II:

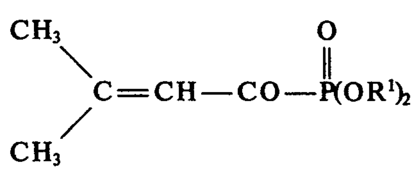

(II)

dans laquelle

$R^1$ a la signification indiquée ci-dessus,

avec des esters d'acide sulfuranylidène-acétique de formule III

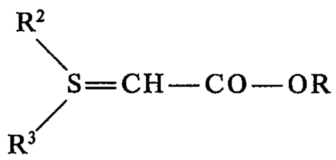

(III)

dans laquelle

R a la signification indiquée plus haut et
$R^2$ et $R^3$ représentent individuellement un alcoyle ou conjointement un alcanediyle (alcoylène),

éventuellement en présence d'un diluant à des températures entre 0 et 100° C.

2. Esters d'acide 1-oxo-3-méthyl-2-butène-phosphonique de formule II

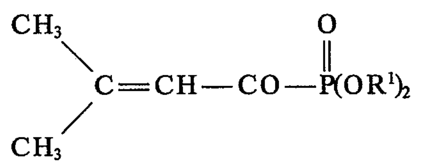

(II)

dans laquelle

$R^1$ représente un alcoyle ou phényle ou bien les deux radicaux $R^1$ représentent ensemble un alcanediyle (alcoylène) à chaîne droite ou ramifiée.

3. Procédé de fabrication d'esters d'acide 1-oxo-3-méthyl-2-butène-phosphonique de formule II, caractérisé en ce que d'une maniée connue en ellemême on fait réagir du chlorure de $\beta,\beta$-diméthylacrylyle de formule IV

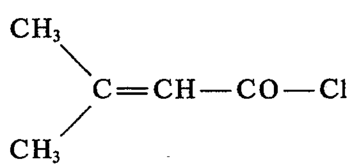

(IV)

avec des esters d'acide phosphoreux de formule V

$$R^4O-P(OR^1)_2 \qquad (V)$$

dans laquelle

$R^1$ a la signification indiquée ci-dessus et
$R^4$ représente un méthyle ou éthyle,

éventuellement en présence d'un diluant à des températures entre 0 et 100° C.